# EUROPÄISCHE PATENTANMELDUNG

(11) **EP 1 157 685 A2**
(43) Veröffentlichungstag der Anmeldung: **28.11.2001**
(21) Anmeldenummer: 01111393.3
(22) Anmeldetag: 10.05.2001
(51) Int. Cl.: A61K 7/32

(54) **Desodorierende Zubereitungen mit Mono- und Oligoglycerinmonocarbonsäuremonoestern, Arylverbindungen und Glycerylethern**

(30) Priorität: 20.05.2000 DE 10025123
(71) Anmelder: Beiersdorf AG, 20245 Hamburg (DE)
(72) Erfinder: Maurer, Peter, 22880 Wedel (DE); Traupe, Bernd, 22457 Hamburg (DE); Petersen, Manuela, 20253 Hamburg (DE); Hallmann, Martina, 24616 Bredstedt (DE); Jacobs, Karin, 20535 Hamburg (DE)

(57) **Zusammenfassung**

Wirkstoffkombinationen, umfassend
(I) eine oder mehrere Substanzen, gewählt aus der Gruppe der Mono- und Oligoglycerinmonocarbonsäure-monoester,
(II) eine oder mehrere Arylverbindungen
(III) einen oder mehrere Glycerylether.

## Beschreibung

Die vorliegende Erfindung betrifft kosmetische Desodorantien.

Kosmetische Desodorantien dienen dazu, Körpergeruch zu beseitigen, der entsteht, wenn der an sich geruchlose frische Schweiß durch Mikroorganismen zersetzt wird. Den handelsüblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Der Schweißfluss selbst wird dadurch nicht beeinflusst, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht. Dies bemerkt schon Plautus (244 - 184 v.u.Z.) in seiner "Gespenstergeschichte" ("Mostellaria", 1. Aufzug, 3.Auftritt: "ubi sese sudor cum unguentis consociavit, ilico itidem olent, quasi cum una multa iura confudit cocus. quid olant nescias, nisi id unum, ut male olere intellegas.")

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Zubereitungen, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachlässt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschenderweise gefunden, und darin liegt die Lösung all dieser Aufgaben, daß Wirkstoffkombinationen, umfassend
(I) eine oder mehrere Substanzen, gewählt aus der Gruppe der Mono- und Oligoglycerin-monocarbonsäure-monoester,
(II) eine oder mehrere Arylverbindungen der allgemeinen Strukturformel 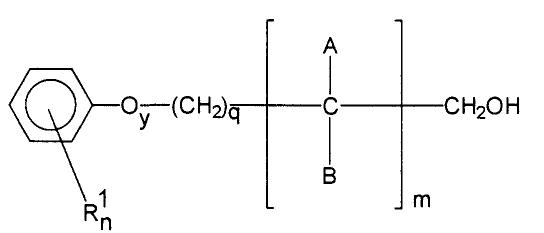 wobei R¹ darstellen kann: H, CH₃, OCH₃, NH₂, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5.
   Der Index m kann Werte von 1 - 10 annehmen, der Index y kann die Werte 0 oder 1 annehmen, der Index q kann Werte von 0 - 10 annehmen, A und B stellen unabhängig voneinander dar H, OH, sowie verzweigte und unverzweigte Alkylreste mit 1 - 10 Kohlenstoffatomen,
(III) einen oder mehrere Glycerylether der allgemeinen Strukturformel 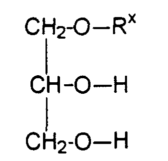 in der R^{x} eine verzweigte oder unverzweigte C₆-C₁₈-Alkylgruppe ist, wobei die Alkylgruppe mit einer oder mehreren Hydroxy- und/oder C₁-C₄-Alkoxygruppe(n) substituiert und/oder die Alkylkette durch bis zu vier Sauerstoffatome unterbrochen sein kann, d.h. Alkylenoxygruppen wie Ethylenoxy- und Propylenoxygruppen enthalten kann,
   sowie insbesondere kosmetische Desodorantien oder gegen Fußgeruch wirksame Zubereitungen, solche Wirkstoffkombinationen enthaltend, den Nachteilen des Standes der Technik abhelfen.

Die erfindungsgemäßen Monoglycerinmonoester werden durch die allgemeine Formel 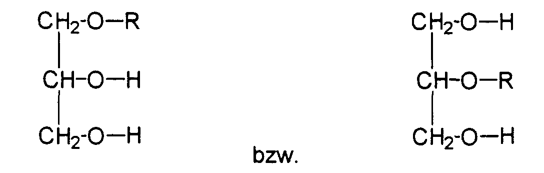 wiedergegeben, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estern zugrundeliegenden Fettsäuren bzw. Monocarbonsäuren sind die

| | | |
|---|---|---|
| Hexansäure | (Capronsäure) | (R= -C₅H₁₁), |
| Heptansäure | (Önanthsäure) | (R= -C₆H₁₃), |
| Octansäure | (Caprylsäure) | (R= -C₇H₁₅), |
| Nonansäure | (Pelargonsäure) | (R= -C₈H₁₇), |
| Decansäure | (Caprinsäure) | (R= -C₉H₁₉), |
| Undecansäure | | (R= -C₁₀H₂₁), |
| 10-Undecensäure | (Undecylensäure) | (R= -C₁₀H₁₉), |
| Dodecansäure | (Laurinsäure) | (R= -C₁₁H₂₃), |
| Tridecansäure | | (R= -C₁₂H₂₅), |
| Tetradecansäure | (Myristinsäure) | (R= -C₁₃H₂₇), |
| Pentadecansäure | | (R= -C₁₄H₂₉), |
| Hexadecansäure | (Palmitinsäure) | (R= -C₁₅H₃₁), |
| Heptadecansäure | (Margarinsäure) | (R= -C₁₆H₃₃), |
| Octadecansäure | (Stearinsäure) | (R= -C₁₇H₃₅). |

In dieser Schrift, insbesondere in den Beispielen, wird das Kürzel GMCy für Glycerinmonocaprylat und das Kürzel GMC für Glycerinmonocaprinat verwendet.

Bei den in 1-Position des Glycerins veresterten Glycerinestern ist die 2-Position ein A-symmetriezentrum. Erfindungsgemäß aktiv und gleichermaßen von Vorteil sind die 2S-und die 2R-Konfiguration.

Es hat sich als günstig herausgestellt, racemische Gemische der Stereoisomeren zu verwenden.

In den dermatologischen Zubereitungen beträgt der Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Erfindungsgemäß liegen die Di- bzw. Triglycerineinheiten der erfindungsgemäßen Diglycerin-monocarbonsäure-monoester bzw. Triglycerin-monocarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäuremonoester des Diglycerins sind bevorzugt durch folgende Struktur gekennzeichnet (Substitutionspositionen angegeben): 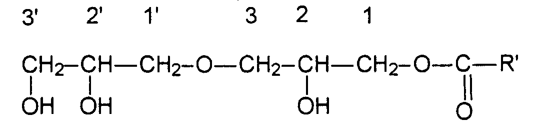 wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäuremonoester des Triglycerins sind bevorzugt durch folgende Struktur gekennzeichnet (Substitutionspositionen angegeben): 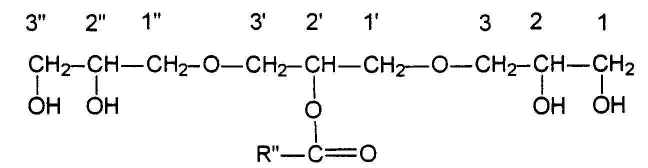 wobei R" einen Kohlenwasserstoffrest, vorteilhaft einen verzweigten oder unverzweigten Alkyl- oder Alkenylrest Alkylrest von 5 bis 17 C-Atomen darstellt.

Die diesen Estem zugrundeliegenden Monocarbonsäuren sind die

| | | |
|---|---|---|
| Hexansäure | (Capronsäure) | (R' bzw. R"= -C₅H₁₁), |
| Heptansäure | (Önanthsäure) | (R' bzw. R"= -C₆H₁₃), |
| Octansäure | (Caprylsäure) | (R' bzw. R"= -C₇H₁₅), |
| Nonansäure | (Pelargonsäure) | (R' bzw. R"= -C₈H₁₇), |
| Decansäure | (Caprinsäure) | (R' bzw. R"= -C₉H₁₉), |
| Undecansäure | | (R' bzw. R"= -C₁₀H₂₁), |
| 10-Undecensäure | (Undecylensäure) | (R' bzw. R"= -C₁₀H₁₉), |
| Dodecansäure | (Laurinsäure) | (R' bzw. R"= -C₁₁H₂₃), |
| Tridecansäure | | (R' bzw. R"= -C₁₂H₂₅), |
| Tetradecansäure | (Myristinsäure) | (R' bzw. R"= -C₁₃H₂₇), |
| Pentadecansäure | | (R' bzw. R"= -C₁₄H₂₉), |
| Hexadecansäure | (Palmitinsäure) | (R' bzw. R"= -C₁₅H₃₁), |
| Heptadecansäure | (Margarinsäure) | (R' bzw. R"= -C₁₆H₃₃), |
| Octadecansäure | (Stearinsäure) | (R' bzw. R"= -C₁₇H₃₅). |

Besonders günstig werden R' und R" gewählt aus der Gruppe der unverzweigten Alkylreste mit ungeraden C-Zahlen, insbesondere mit 9, 11 und 13 C-Atomen.

Im allgemeinen sind die Monocarbonsäuremonoester des Diglycerins denen des Triglycerins bevorzugt.

Ganz besonders günstig sind

| | | |
|---|---|---|
| Diglycerinmonocaprinat | (DMC) | R' = 9 |
| Triglycerinmonolaurat | (TML) | R" = 11 |
| Diglycerinmonolaurat | (DML) | R' = 11 |
| Triglycerinmonomyristat | (TMM) | R" = 13. |

Als bevorzugter erfindungsgemäßer Monocarbonsäuremonoester des Diglycerins hat sich das Diglycerinmonocaprinat (DMC) erwiesen.

Die erfindungsgemäßen Monocarbonsäuremonoester des Diglycerins liegen bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestem des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestem sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2"-Position Asymmetriezentren. Erfindungsgemäß aktiv und gleichermaßen von Vorteil sind die 2S2'S2"S-, die 2R2'S2"S-, die 2S2'R2"S-, die 2R2'R2"S-, die 2S2'S2"R, die 2R2'S2"R-, die 2S2'R2"R- und die 2R2'R2"R-Konfiguration.

Es hat sich als günstig herausgestellt, racemische Gemische der Stereoisomeren zu verwenden.

Erfindungsgemäß bevorzugte Arylverbindungen werden gewählt aus der Gruppe Phenoxyethanol, Anisalkohol, 2-Methyl-5-phenyl-pentan-1-ol, 2-Methyl-4-phenylbutan-2-ol.

Phenoxyethanol ist gekennzeichnet durch die Strukturformel

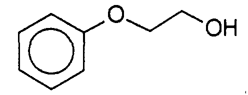

Anisalkohol ist gekennzeichnet durch die Strukturformel

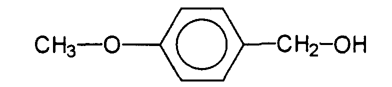

2-Methyl-5-phenyl-pentan-1-ol ist gekennzeichnet durch die Strukturformel

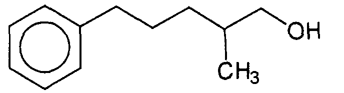

2-Methyl-4-phenylbutan-2-ol ist gekennzeichnet durch die Strukturformel

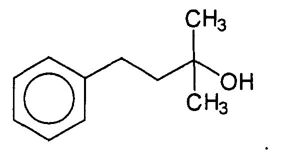

Besonders vorteilhaft werden der oder die Glycerylether gewählt aus der Gruppe Hexoxyglycerin und Octoxyglycerin.

Es ist zwar aus der DE-OS 195 41 967 bekannt, Partialglyceride und arylierte Alkohole als Bestandteil kosmetischer Desodorantien einzusetzen. Der Stand der Technik konnte jedoch keinen Hinweis auf die erfindungsgemäßen, synergistisch wirkenden Wirkstoffkombinationen mit Glycerylethem geben.

Die erfindungsgemäßen Zubereitungen sind besonders vorteilhaft dadurch gekennzeichnet, daß der oder die Monocarbonsäure-Monoester des Mono-, Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Die erfindungsgemäßen Zubereitungen sind weiterhin besonders vorteilhaft dadurch gekennzeichnet, daß die Arylverbindung oder die Arylverbindungen in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Die erfindungsgemäßen Zubereitungen sind weiterhin besonders vorteilhaft dadurch gekennzeichnet, daß der oder die Glycerylether in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Erfindungsgemäße Zubereitungen, die erfindungsgemäßen Wirkstoffkombinationen enthaltend, sind besonders vorteilhaft dadurch gekennzeichnet, daß die erfindungsgemäßen Wirkstoffkombinationen in Konzentrationen von 0,05 - 10,00 Gew.-%, bevorzugt 0,1 - 5,0 Gew.-%, vorliegen, jeweils bezogen auf das Gesamtgewicht der Zubereitungen.

Entsprechend der erfindungsgemäßen Verwendung können die kosmetischen Desodorantien in Form von Aerosolen, also aus Aerosolbehältem, Quetschflaschen oder durch eine Pumpvorrichtung versprühbaren Präparaten vorliegen oder in Form von mittels Rollon-Vorrichtungen auftragbaren flüssigen Zusammensetzungen, als Deo-Stifte (Deo-Sticks) und in Form von aus normalen Flaschen und Behältern auftragbaren W/O- oder O/W-Emulsionen, z.B. Crèmes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Als übliche kosmetische Trägerstoffe zur Herstellung der desodorierenden Zubereitungen gemäß der erfindungsgemäßen Verwendung können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butylund Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoäharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder-monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethyihexyiisostearat.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Camaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die erfindungsgemäße Wirkstoff-Kombination ist besonders für den Einsatz in alkoholischen Aerosolzubereitungen geeignet, da sie eine hohe Deowirkung gewährleistet und daher auf den Einsatz von üblicherweise auch zum Zwecke der Desodorierung eingesetzten Aluminiumsalze verzichtet, die für rein alkoholische Anwendungsformen relativ schlecht geeignet sind.

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Darüber hinaus lassen sich die erfindungsgemäßen Formulierungen sehr vorteilhaft in Form von getränkten Tüchern anwenden, die aus den unterschiedlichsten Materialien wie z.B. Papier oder natürlichen bzw. synthetischen Fasern bestehen können. Als Tränkungsmedium eigenen sich hierbei grundsätzlich alle flüssigen Formulierungen. Die Tücher können einzeln verpackt bzw. als wiederverschließbare Mehrfachpackung dargeboten werden.

Es folgen vorteilhafte Ausführungsbeispiele der vorliegenden Erfindung.

### Beispiel

| | Gew.-% |
|---|---|
| Propylenglycol | 3,22 |
| DMC | 1,00 |
| Octoxyglycerin | 1,00 |
| Methylphenylbutanol | 0,80 |
| Octyldodecanol | 0,16 |
| Parfum | q.s. |
| Alkohol denat. | ad 100,00 |

## Patentansprüche

1. Wirkstoffkombinationen, umfassend
(I) eine oder mehrere Substanzen, gewählt aus der Gruppe der Mono- und Oligoglycerinmonocarbonsäure-monoester,
(II) eine oder mehrere Arylverbindungen der allgemeinen Strukturformel 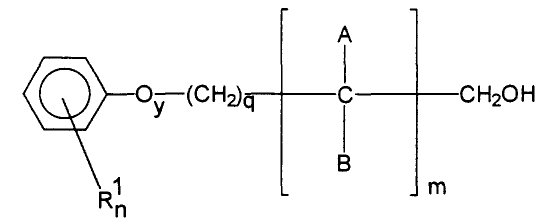 wobei R¹ darstellen kann: H, CH₃, OCH₃, NH₂, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5.
Der Index m kann Werte von 1 - 10 annehmen, der Index y kann die Werte 0 oder 1 annehmen, der Index q kann Werte von 0 - 10 annehmen, A und B stellen unabhängig voneinander dar H, OH, sowie verzweigte und unverzweigte Alkylreste mit 1 - 10 Kohlenstoffatomen,
(III) einen oder mehrere Glycerylether der allgemeinen Strukturformel 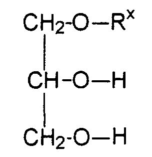 in der Rx eine verzweigte oder unverzweigte C6-C18-Alkylgruppe ist, wobei die
Alkylgruppe mit einer oder mehreren Hydroxy- und/oder C1-C4-Alkoxygruppe(n) substituiert und/oder die Alkylkette durch bis zu vier Sauerstoffatome unterbrochen sein kann, d.h. Alkylenoxygruppen wie Ethylenoxy- und Propylenoxygruppen enthalten kann.

2. Kosmetische Desodorantien oder gegen Fußgeruch wirksame Zubereitungen, Wirkstoffkombinationen nach Anspruch 1 enthaltend.

3. Wirkstoffkombinationen nach Anspruch 1 oder Zubereitungen nach Anspruch 2, **dadurch gekennzeichnet, daß** als Mono- und Oligoglycerinmonocarbonsäure-monoester das Diglycerylmonocaprat gewählt wird.

4. Wirkstoffkombinationen nach Anspruch 1 oder Zubereitungen nach Anspruch 2, **dadurch gekennzeichnet, daß** die Arylverbindungen gewählt werden aus der Gruppe Phenoxyethanol, Anisalkohol, 2-Methyl-5-phenyl-pentan-1-ol, 2-Methyl-4-phenylbutan-2-ol.

5. Wirkstoffkombinationen nach Anspruch 1 oder Zubereitungen nach Anspruch 2, **dadurch gekennzeichnet, daß** der oder die Glycerylether gewählt werden aus der Gruppe Hexoxyglycerin und Octoxyglycerin.

6. Wirkstoffkombinationen nach Anspruch 1 oder Zubereitungen nach Anspruch 2, **dadurch gekennzeichnet, daß** der oder die Mono- oder Dicarbonsäure-Monoester des Mono-, Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

7. Wirkstoffkombinationen nach Anspruch 1 oder Zubereitungen nach Anspruch 2, **dadurch gekennzeichnet, daß** die Arylverbindung oder die Arylverbindungen in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

8. Wirkstoffkombinationen nach Anspruch 1 oder Zubereitungen nach Anspruch 2, **dadurch gekennzeichnet, daß** der oder die Glycerylether in Konzentrationen von 0,01-10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.
